# EUROPÄISCHE PATENTSCHRIFT

(11) **EP 3 554 427 B1**
(45) Veröffentlichungstag und Bekanntmachung des Hinweises auf die Patenterteilung: **09.08.2023**
(21) Anmeldenummer: 17818079.0
(22) Anmeldetag: 06.12.2017
(51) Int. Cl.: A61F 2/44, A61F 2/30

(54) **BANDSCHEIBENPROTHESE UND VERFAHREN ZUR HERSTELLUNG EINER BANDSCHEIBENPROTHESE**
INTERVERTEBRAL DISK PROSTHESIS AND METHOD FOR PRODUCING AN INTERVERTEBRAL DISK PROSTHESIS
PROTHÈSE DE DISQUE INTERVERTÉBRAL ET PROCÉDÉ DE PRODUCTION D'UNE PROTHÈSE DE DISQUE INTERVERTÉBRAL

(30) Priorität: 19.12.2016 DE 102016124877
(43) Veröffentlichungstag der Anmeldung: 23.10.2019
(73) Patentinhaber: NGMedical GmbH, 66620 Nonnweiler (DE)
(72) Erfinder: KRÄMER, Sina, 66538 Neunkirchen (DE); WENZEL, Rudolf, 54422 Züsch (DE)
(74) Vertreter: Meissner Bolte Partnerschaft mbB
(86) Internationale Anmeldenummer: PCT/EP2017/081674
(87) Internationale Veröffentlichungsnummer: WO 2018/114334

(56) Entgegenhaltungen:
- WO-A1-2009/055796
- WO-A1-2012/045340
- US-A1- 2008 319 548
- US-A1- 2012 150 298
- US-A1- 2014 350 681

## Beschreibung

Die Erfindung betrifft eine Bandscheibenprothese, umfassend eine kaudale Platte, eine kraniale Platte und einen zwischen der kaudalen Platte und der kranialen Platte ausgebildeten elastischen Kern, gemäß den Patentansprüchen 1 oder 2.

Des Weiteren betrifft die Erfindung ein Verfahren zur Herstellung einer Bandscheibenprothese, insbesondere einer erfindungsgemäßen Bandscheibenprothese, gemäß Patentanspruch 8.

Bandscheibenprothesen sind bewegungserhaltende Implantate, die den Höhenverlust bei Bandscheibenvorfällen ausgleichen und die natürliche Funktion und Beweglichkeit der Wirbelsäule, insbesondere einer Halswirbelsäule, weitgehend wiederherstellen. Die vorwiegend verwendeten Kugelgelenkprothesen beschränken die Bewegung nicht. Die Bewegung wird durch die Bänder und Muskeln limitiert. Kugelgelenkprothesen beruhen meist auf einer überlagerten Translationsbewegung, bei der der Kern selbst gleitet. Aufgrund der Limitation der Bewegung der Kugelgelenksprothesen durch die Bänder und Muskeln werden diese stark belastet, so dass Kopf- und Nackenschmerzen auftreten können.

Eine bei Bandscheibenprothesen wünschenswerte Dämpfung muss die Kopflast beim Gehen aufnehmen und verteilen, so dass diese Kopflast nicht schlagartig auf die Wirbel einwirkt. Klinische Folgen fehlender Dämpfung sind Lastspitzen in der Berührungsfläche zwischen der Prothese und dem Wirbel, welche die Fixierung der Bandscheibenprothese beeinträchtigen. Ein Schlag auf bekannte Bandscheibenprothesen führt zu höheren Dislokations- und Versagensrisiken, wobei des Weiteren eine erhöhte Belastung auf alle Wirbel zu beobachten ist.

Die Patentschriften US 2012/150298 A1 und WO 2009/055796 A1 zeigen Beispiele von Bandscheibenprothesen, die eine kaudale Platte, eine kraniale Platte und einen zwischen der kaudalen Platte und der kranialen Platte ausgebildeten elastischen Kern offenbaren. In den jeweiligen Beispielen weist die kaudale Platte auf der zur kranialen Platte weisenden Seite eine Ausnehmung auf, wobei der Kern formschlüssig mit der Ausnehmung der kaudalen Platte verbunden ist und die kraniale Platte nicht mit dem Kern verbunden ist. Darüber hinaus weist die kraniale Platte mindestens einen Führungsstift auf, der in eine Führungskontur des Kerns beweglich eingreift.

Aus dem Vorgenannten ist es daher die Aufgabe der vorliegenden Erfindung, eine weiterentwickelte Bandscheibenprothese, insbesondere eine weiterentwickelte zervikale Bandscheibenprothese, anzugeben, welche die aufgezeigten Nachteile bisher bekannter Bandscheibenprothesen überwindet. Insbesondere soll eine Bandscheibenprothese zur Verfügung gestellt werden, die eine natürliche Bewegung der Halswirbelsäule mit sechs Freiheitsgraden ermöglicht.

Des Weiteren ist es Aufgabe der vorliegenden Erfindung, ein weiterentwickeltes Verfahren zur Herstellung einer Bandscheibenprothese, insbesondere zur Herstellung einer erfindungsgemäßen Bandscheibenprothese, anzugeben.

Die Lösung der Aufgabe erfolgt durch eine Bandscheibenprothese, die eine kaudale Platte, eine kraniale Platte und einen zwischen der kaudalen Platte und der kranialen Platte ausgebildeten elastischen Kern aufweist, gemäß Merkmalskombination der Patentansprüche 1 oder 2.

Hinsichtlich des Verfahrens erfolgt die Lösung der Aufgabe durch ein Verfahren zur Herstellung einer Bandscheibenprothese, insbesondere einer erfindungsgemäßen Bandscheibenprothese, gemäß Merkmalskombination des Patentanspruches 11. Die jeweiligen Unteransprüche stellen mindestens zweckmäßige Ausgestaltungen und Weiterbildungen dar.

Erfindungsgemäß weist die kaudale Platte auf der zur kranialen Platte weisenden Seite eine Ausnehmung auf, wobei der Kern formschlüssig mit der Ausnehmung der kaudalen Platte verbunden ist.

Erfindungsgemäß ist der Kern haltend, nämlich formschlüssig, mit der kaudalen Platte verbunden. Insbesondere sind keine weiteren Zusatzwerkstoffe oder Zusatzbauteile zur Verbindung der kaudalen Platte mit dem elastischen Kern notwendig. Die kraniale Platte ist hingegen nicht mit dem elastischen Kern verbunden. Vielmehr liegt die kraniale Platte auf dem elastischen Kern auf. Aufgrund dessen wird eine Prothese aus zwei Platten und einem elastischen Kern zur Verfügung gestellt, die eine natürliche Bewegung der Wirbelsäule, insbesondere der Halswirbelsäule, mit sechs Freiheitsgraden ermöglicht.

Bei den sechs Freiheitsgraden handelt es sich um eine Flexion, eine Extension sowie eine Seitneigung in axialer Richtung und um eine Rotation, eine Translation sowie eine Steifigkeit in kaudal-kranialer Richtung. Dabei ist der Bewegungsumfang in jede Richtung unterschiedlich und dem physiologischen Bewegungsumfang (range of motion) angepasst.

In einer bevorzugten Ausführungsform der Erfindung weist die Ausnehmung zumindest abschnittsweise eine Hinterschneidung auf.

Der Kern ist vorzugsweise in die Ausnehmung eingespritzt oder eingegossen, insbesondere mittels eines Urformverfahrens, besonders bevorzugt mittels eines Kunststoffspritzgießverfahrens oder Vakuumgussverfahrens eingespritzt oder eingegossen. Die Einspritzung bzw. das Eingießen eines Kerns in die Ausnehmung, insbesondere in eine Ausnehmung, die zumindest abschnittsweise eine Hinterschneidung aufweist, bildet eine formschlüssige Verbindung. Demnach ist es nicht notwendig, den Kern mit der kaudalen Platte zu verkleben. Des Weiteren ist es ebenfalls nicht notwendig, den Kern mit weiteren Hilfsmitteln, wie beispielsweise Fäden, mit der kaudalen Platte zu verbinden.

Der Kern weist in einer besonders bevorzugten Ausführungsform viskoelastische Eigenschaften auf. Es ist möglich, dass der Kern aus einem Elastomer, insbesondere aus Silikon oder PCU, besteht. Die kraniale Platte ist nicht mit dem Kern verbunden. Die kraniale Platte kann allerdings am Kern anliegen bzw. auf dem Kern aufliegen. Das heißt, dass eine Berührung des Kerns durch die kraniale Platte keiner festen Verbindung entspricht.

Die kraniale Platte weist erfindungsgemäß mindestens einen Führungsstift auf, der in eine Führungskontur des Kerns beweglich eingreift. Als Führungsstift sind auch Führungselemente mit größerem Durchmesser zu verstehen. Ein Führungsstift beschreibt mit anderen Worten ein Führungselement, das von der zum elastischen Kern weisenden Seite der kranialen Platte absteht.

Der Kern weist eine, insbesondere komplementär zur Geometrie des Führungsstifts ausgebildete, Führungskontur auf. Die Führungskontur kann beispielsweise als Ausnehmung bzw. Materialaussparung ausgebildet sein.

Der Führungsstift kann erfindungsgemäß im Querschnitt im Wesentlichen eine Kreuzform aufweisen, wobei die Führungskontur des Kerns vorzugsweise als eine komplementär zur Kreuzform ausgebildete Ausnehmung ausgebildet ist. Bei der Kreuzform des Führungsstiftes kann es sich auch um ein derartiges Kreuz handeln, das an den axialen Enden jeweils einen Quersteg aufweist. Die Ausnehmung des Kerns, die die Führungskontur bildet, weist eine hierzu komplementäre Form mit ebenfalls ausgebildeten Querstegen auf.

Vorzugsweise ist die Ausnehmung bzw. Aussparung größer als der Querschnitt des Führungsstiftes ausgebildet. Dadurch wird insbesondere eine freie Translation ermöglicht. Die Dimension des Bewegungsumfangs in der Neutralzone wird über die Geometrie der Ausnehmung bzw. Materialaussparung abgebildet. Die geometrische Form der Ausnehmung bzw. die geometrische Form im Kern ermöglicht im Zusammenspiel mit der kranialen Platte eine Bewegung, wobei die Platte in dem elastischen Kern geführt wird. Mit anderen Worten greift mindestens ein Abschnitt der kranialen Platte in den Kern, insbesondere in eine Führungskontur des Kerns, ein.

In einer weiteren Ausführungsform der Erfindung ist es möglich, dass die kraniale Platte mindestens zwei Führungsstifte aufweist, wobei die Führungskontur des Kerns als mindestens zwei gebogene, insbesondere nierenförmige, Ausnehmungen ausgebildet ist. Bei den beiden Führungsstiften handelt es sich vorzugsweise um Führungsstifte mit kreisrundem Querschnitt.

Die beiden gebogenen Ausnehmungen können sowohl als Vertiefungen als auch als vollständige Ausnehmungen ausgebildet sein. Insbesondere sind die Ausnehmungen als gebogene, insbesondere nierenförmige, Langlöcher ausgebildet. Vorzugsweise liegen die mindestens zwei gebogenen, insbesondere nierenförmigen, Ausnehmungen auf einer gemeinsamen Ellipse. Des Weiteren ist es möglich, dass der Kern eine ellipsenförmige Nut aufweist, wobei in der ellipsenförmigen Nut die mindestens zwei gebogenen, insbesondere nierenförmigen, Ausnehmungen ausgebildet sind. Die ellipsenförmige Nut begrenzt eine mittige Ellipse. Die mittige Ellipse entspricht der Kernmitte.

Der Kern kann des Weiteren auf der zur kranialen Platte weisenden Seite gewölbt ausgebildet sein. Vorzugsweise ist der Kern auf der erwähnten Seite sowohl in Längsrichtung als auch in Querrichtung gewölbt. Dies ermöglicht ein besonders einfaches Gleiten der kranialen Platte auf dem elastischen Kern.

Die zum Knochen weisende Seite der kaudalen Platte und/oder die zum Knochen weisende Seite der kranialen Platte kann eine Verzahnung und/oder eine Riffelung aufweisen. Eine Verzahnung und/oder Riffelung dient zur Primärverankerung der kaudalen Platte und/oder der kranialen Platte an dem Knochen, insbesondere an den Wirbeln.

In einer besonders bevorzugten Ausführungsform der Erfindung ist die Hinterschneidung in der Ausnehmung der kaudalen Platte vollumfänglich ausgebildet. Dies ermöglicht eine besonders gute Befestigung des elastischen Kerns in der Ausnehmung der kaudalen Platte. Die Ausnehmung weist insbesondere eine rechteckige Form auf.

Der elastische, insbesondere viskoelastische, Kern muss abhängig von der Shore-Härte derart stabil sein, dass die mechanische Führung der (lastfreien) Bewegung der kranialen Platte in einer Neutralzone erlaubt wird. Dies betrifft den physiologischen Bewegungsbereich. In der Neutralzone wird die Bewegung mechanisch geführt und ist mit geringster Last bzw. Gegenlast durchführbar. Die mechanische Führung der Bewegung erfolgt über die Geometrie des Kerns.

Am Maximum des physiologischen Bewegungsbereiches erfährt die Bewegung einen progressiven Kraftanstieg. Dies wird durch die Geometrie und durch das viskoelastische Verhalten des Kerns bedingt. Das heißt, je weiter das Wirbelsegment aus der Neutrallage bewegt wird, desto größer wird die Kraft. Ein harter Anschlag wird aufgrund der erfindungsgemäßen Ausbildung der Bandscheibenprothese vermieden. Der Bandapparat sowie die Muskeln der Wirbelsäule werden aufgrund der erfindungsgemäßen Bandscheibenprothese entlastet.

Aufgrund der Geometrie der Führungskontur, insbesondere der im Kern ausgebildeten Ausnehmung, und dem dazu invers gestalteten Führungsstift der kranialen Platte wird eine Dislokation der Bandscheibenprothese verhindert. Eine Dislokation beschreibt das Verrutschen der kranialen Platte zum Kern.

Der Bewegungsumfang der erfindungsgemäßen Bandscheibenprothese ist in jede Dimension einzeln durch die geometrische Ausführung des elastischen, insbesondere viskoelastischen, Kerns auf das physiologische Maß beschränkt und definierbar. Aus dem Stand der Technik existieren hierzu lediglich dämpfende Prothesen mit einem progressiven Kraftanstieg, deren Bewegungsumfang jedoch in jede Richtung gleich ist.

Der elastische Kern nimmt im implantierten Zustand die Kopflast (follower load) auf und dämpft zusätzlich bei Bewegung die Kraft. Mit anderen Worten senkt der elastische Kern bei Bewegung die Kraft ab. In axialer Richtung wird somit eine gewisse Steifigkeit realisiert. Durch die Höhenveränderung des elastischen Kerns wird somit eine axiale Stoßlast abgefangen.

Mit Hilfe der Verzahnung und/oder Riffelung der kaudalen Platte und/oder der kranialen Platte wird eine Primärstabilität der eingesetzten Bandscheibenprothese gewährleistet. Die Verzahnung und/oder Riffelung begünstigen das Verwachsen der kranialen Platte und/oder der kaudalen Platte mit dem Knochen. Der finale Halt der implantierten Bandscheibenprothese wird durch sekundäres Verwachsen mit dem Wirbelknochen erreicht.

Die kraniale Platte und/oder die kaudale Platte kann/können aus Metall und/oder Keramik und/oder Kunststoff, insbesondere Polyetheretherketon (PEEK), gebildet sein. Es ist möglich, unterschiedliche Materialpaarung für die kraniale und kaudale Platte zu verwenden.

Die Konstruktion der Bandscheibenprothese ermöglicht es, dass die Bandscheibenprothese derart anatomisch angepasst ist, dass diese besonders vorteilhaft in das Bandscheibenfach eingesetzt werden kann, an dem Uncovertebralgelenk anliegt und die natürliche Lordose unterstützt.

Vorab ist kein spezielles Präparieren der Knochensubstanz durch Fräsen oder Einschlagen von Kielen notwendig. Dies vereinfacht die Operationstechnik, so dass das Risiko einer iatrogenen Schädigung wie z.B. Nervenschädigungen oder Abbruch von Knochenfragmenten oder der Gefahr der Prothesen-Dislokation durch fehlende Wirbelteile vermindert werden.

In einer besonders bevorzugten Ausführungsform der Erfindung ist die Bandscheibenprothese als eine zervikale Bandscheibenprothese ausgebildet.

Ein weiterer nebengeordneter Aspekt der Erfindung betrifft ein Verfahren zur Herstellung einer Bandscheibenprothese, insbesondere einer zuvor beschriebenen erfindungsgemäßen Bandscheibenprothese.

Das erfindungsgemäße Verfahren zur Herstellung einer erfindungsgemäßen Bandscheibenprothese beruht auf den Verfahrensschritten:
a) Bereitstellen einer kaudalen Platte mit einer Ausnehmung, die vorzugsweise zumindest abschnittsweise eine Hinterschneidung aufweist,
b) Einspritzen oder Eingießen eines elastischen, insbesondere viskoelastischen, Materials in die Ausnehmung und Bilden eines elastischen, insbesondere viskoelastischen, Kerns.

Vorzugsweise wird die kaudale Platte vor Schritt b) in eine Form, insbesondere in ein Spritzgusswerkzeug, eingelegt.

Vorzugsweise ist die (erfindungsgemäße) kaudale Platte auf der zum Knochen weisenden Seite derart ausgebildet, dass eine Verzahnung und/oder Riffelung nicht vollflächig ausgebildet ist. Insbesondere ist es möglich, dass auf der zum Knochen weisenden Seite der kaudalen Platte ein Randbereich ohne Verzahnung und/oder Riffelung ausgebildet ist, sodass die kaudale Platte zumindest abschnittsweise plan aufliegend in eine Form, insbesondere in ein Spritzgusswerkzeug, eingelegt werden kann. Mit anderen Worten dient der beschriebene Randbereich beispielsweise als Auflagefläche im Spritzgusswerkzeug.

In einer weiteren Ausführungsform des erfindungsgemäßen Verfahrens weist die (erfindungsgemäße) kaudale Platte auf der zum Kern weisenden Seite ebenfalls einen Randbereich/Randabschnitt auf, der zur Abdichtung im Werkzeug dient. Insbesondere wird auf den Randbereich/Randabschnitt ein Formwerkzeug aufgelegt, um anschließend den elastischen Kern bilden zu können. Mit anderen Worten dient als Auflagefläche für das Formwerkzeug ein Randbereich/Randabschnitt der Seite der kaudalen Platte, die im fertig montierten Zustand zum Kern weist. Der Randbereich dient zur Abdichtung im Spritzgusswerkzeug.

Gemäß einem Schritt c) folgt nach dem Bilden eines elastischen, insbesondere viskoelastischen, Kerns, ein Auflegen einer kranialen Platte auf den Kern derart, dass mindestens ein Führungsstift der kranialen Platte in eine Führungskontur des Kerns beweglich eingreift.

Die Erfindung wird im Folgenden anhand mehrerer Ausführungsbeispiele unter Bezugnahme auf die beigefügten, schematischen Zeichnungen näher erläutert.

Darin zeigen:
- Fig. 1a - 1b: mehrere Ansichten auf eine erfindungsgemäße Bandscheibenprothese gemäß einem ersten Ausführungsbeispiel;
- Fig. 2a und 2b: verschiedene Ansichten auf eine erfindungsgemäße Bandscheibenprothese gemäß einem zweiten Ausführungsbeispiel;
- Fig. 3a - 3c: mehrere Ansichten auf eine erfindungsgemäße Bandscheibenprothese gemäß einem dritten Ausführungsbeispiel; und
- Fig. 4: eine Explosionsdarstellung einer erfindungsgemäßen Bandscheibenprothese gemäß einem vierten Ausführungsbeispiel.

In der nachfolgenden Beschreibung werden für gleiche und gleichwirkende Teile dieselben Bezugsziffern verwendet.

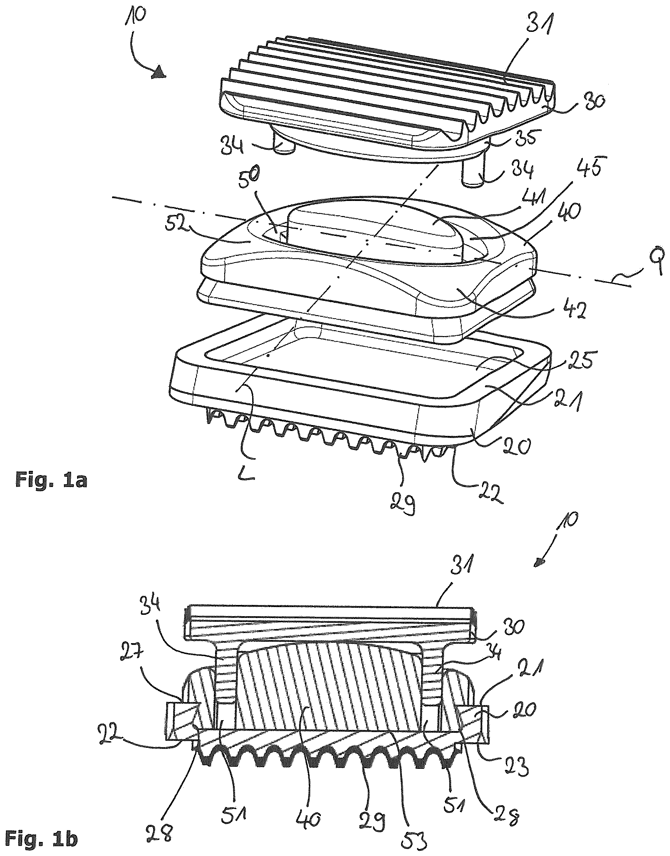

In den Fig. 1a und 1b ist eine Bandscheibenprothese 10 gemäß einem ersten Ausführungsbeispiel dargestellt. In Fig. 1a ist eine Explosionsdarstellung der einzelnen Bestandteile der Bandscheibenprothese 10 dargestellt. In Fig. 1b ist hingegen ein Querschnitt durch die Bandscheibenprothese 10 dargestellt.

Es ist darauf hinzuweisen, dass die einzelnen Elemente der Bandscheibenprothese 10 im tatsächlich fixierten bzw. hergestellten Zustand nicht wie in Fig. 1a dargestellt auseinandergenommen werden können. Dies ist vor allen Dingen aufgrund des erfindungsgemäßen Herstellungsverfahrens nicht ohne weiteres möglich.

Bei der Bandscheibenprothese 10 handelt es sich beispielsweise um eine zervikale Bandscheibenprothese. Diese umfasst eine kaudale Platte 20, eine kraniale Platte 30 und einen zwischen der kaudalen Platte 20 und der kranialen Platte 30 ausgebildeten viskoelastischen Kern 40. Die kaudale Platte 20 weist auf der zur kranialen Platte 30 weisenden Seite 21 eine Ausnehmung 25 auf. Wie insbesondere in Fig. 1b dargestellt ist, weist die Ausnehmung 25 eine Hinterschneidung 28 auf.

Der Kern 40 ist formschlüssig mit der Ausnehmung 25 der kaudalen Platte 20 verbunden. Insbesondere ist der viskoelastische Kern in die Ausnehmung 25 eingespritzt oder eingegossen. Bei dem viskoelastischen Kern handelt es sich um einen Kern aus Elastomer, wie beispielsweise Silikon oder PCU.

Im Rahmen des erfindungsgemäßen Herstellungsverfahrens kann zunächst die kaudale Platte 20 mit der Ausnehmung 25 sowie der Hinterschneidung 28 zur Verfügung gestellt werden. Die kaudale Platte 20 wird beispielsweise in ein Spritzgusswerkzeug eingelegt. Anschließend kann das viskoelastische Material in die kaudale Platte eingegossen oder eingespritzt werden.

Die kaudale Platte 20 weist auf der Unterseite bzw. der zum Knochen weisenden Seite 22 zumindest abschnittsweise eine Riffelung 29 auf. Im dargestellten Beispiel ist die Riffelung 29 nicht über die gesamte Unterseite 22 ausgebildet. Vielmehr wird ein vollumfänglicher Randabschnitt 23 gebildet, der als Auflagefläche im Spritzgusswerkzeug dient. Nach Einlegen der kaudalen Platte 20 in das Spritzgusswerkzeug wird anschließend ein Formwerkzeug aufgelegt, um anschließend den viskoelastischen Kern 40 bilden zu können. Als Auflagefläche für das Formwerkzeug dient hierbei der Randbereich 27 der Seite 21 der kaudalen Platte 20. Der Randbereich 27 dient zur Abdichtung im Spritzgusswerkzeug.

Die Hinterschneidung 28 in der Ausnehmung 25 ist vollumfänglich ausgebildet. Darin wird das viskoelastische Material eingespritzt, ein Kern 40 gebildet und eine formschlüssige Verbindung des Kerns 40 mit der kaudalen Platte 20 hergestellt. Somit wird verhindert, dass das Material aus der kaudalen Platte 20 anschließend entnehmbar ist. Aufgrund der formschlüssigen Verbindung des Kerns 40 mit der Ausnehmung 25 der kaudalen Platte 20 sind keine zusätzlichen Befestigungselemente oder Zusatzwerkstoffe wie z.B. Klebemittel oder Fäden notwendig. Die Montage der Bandscheibenprothese 10 findet somit quasi während der Herstellungsphase statt.

Die kraniale Platte 30 weist auf der Oberseite bzw. der zum Knochen weisenden Seite 31 eine Verzahnung 32 auf. Die kraniale Platte 30 weist des Weiteren zwei Führungsstifte 34 auf. Die Führungsstifte 34 sind auf einem Verbindungsabsatz 35 der kranialen Platte 30 ausgebildet.

Die kraniale Platte 30 ist nicht mit dem Kern verbunden. Die kraniale Platte 30 liegt lediglich auf der zur kaudalen Platte 20 weisenden Seite 33 auf dem Kern 40 auf. Die Führungsstifte 34 greifen in die Führungskontur 50 des viskoelastischen Kerns 40 ein. Die Führungskontur 50 ist im dargestellten Beispiel in Form von zwei Ausnehmungen 51 ausgebildet. Die Ausnehmungen 51 sind im Wesentlichen gebogen ausgebildet. Insbesondere weisen die Ausnehmungen 51 eine Nierenform auf. Demnach ist eine Verdrehung der kranialen Platte 30 in Relation zum elastischen Kern 40 möglich. Die Führungskontur 50 ist als Abschnitt einer ellipsenförmigen Nut 45 ausgebildet.

Der elastische Kern 40 ist des Weiteren gewölbt ausgebildet. Der Kern 40 ist insbesondere auf der zur kranialen Platte 30 weisenden Seite 52 gewölbt ausgebildet. Die Seite 52 ist sowohl in Querrichtung Q als auch in Längsrichtung L gewölbt ausgebildet. Aufgrund der Nut 45, die eine Ellipsenbahn bildet, ist ein elastischer Kern 40 mit einer Kernmitte 41 gebildet, die eine Ellipsenform aufweist und sowohl in Längsrichtung L als auch in Querrichtung Q gewölbt ist. Dies ermöglicht auch eine Kippbewegung der kranialen Platte 30 in Relation zum elastischen Kern 40.

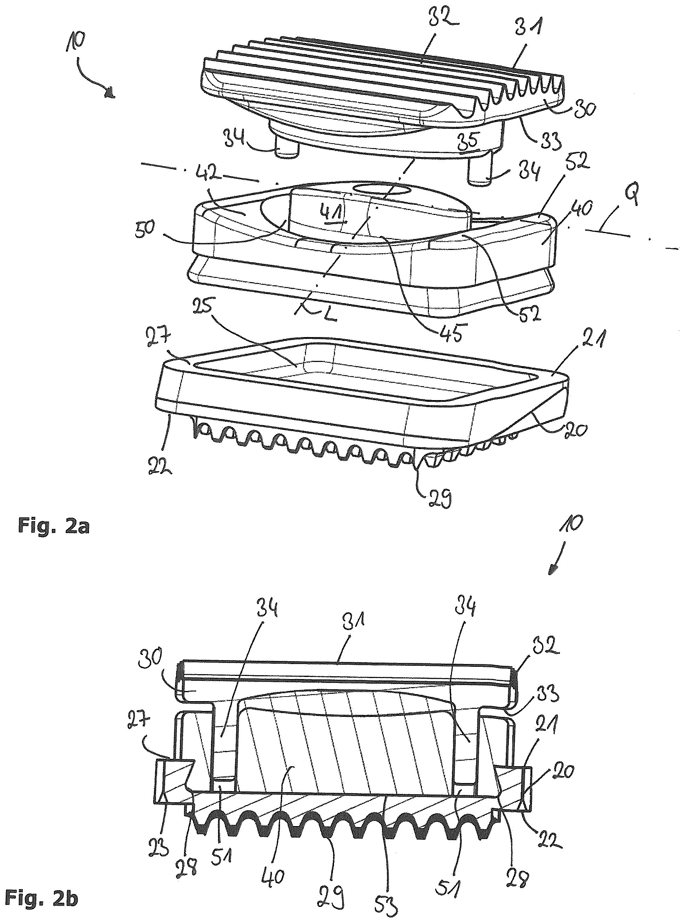

In den Fig. 2a und 2b ist eine zu den Fig. 1a und 1b ähnliche Ausführungsform einer zervikalen Bandscheibenprothese 10 dargestellt. Im Folgenden wird lediglich auf die Unterschiede eingegangen. Diese sind insbesondere im Zusammenhang mit der Ausbildung des elastischen Kerns 40 zu erkennen.

In den Fig. 1a und 1b ist die Seite 52 des Kerns 40 in Querrichtung Q und in Längsrichtung L konvex gewölbt.

In Fig. 2a ist der Kern 40 in mehrere Teilabschnitte unterteilt. Die Kernmitte 41 ist in Querrichtung Q konvex gekrümmt, wohingegen der Bereich außerhalb der Nut 45 in Längsrichtung L konkav gewölbt ist. Dadurch wird eine Kernmitte 41 gebildet, die deutlich über den Umfangsabschnitt 42 des Kerns 40 hervorsteht. Es ergibt sich eine Doppel-Sattel-Funktion.

Die kaudale Platte 20 ist wie bereits in Fig. 1a und 1b dargelegt, mit einer Ausnehmung 27 und einer vollumfänglichen Hinterschneidung 28 ausgebildet.

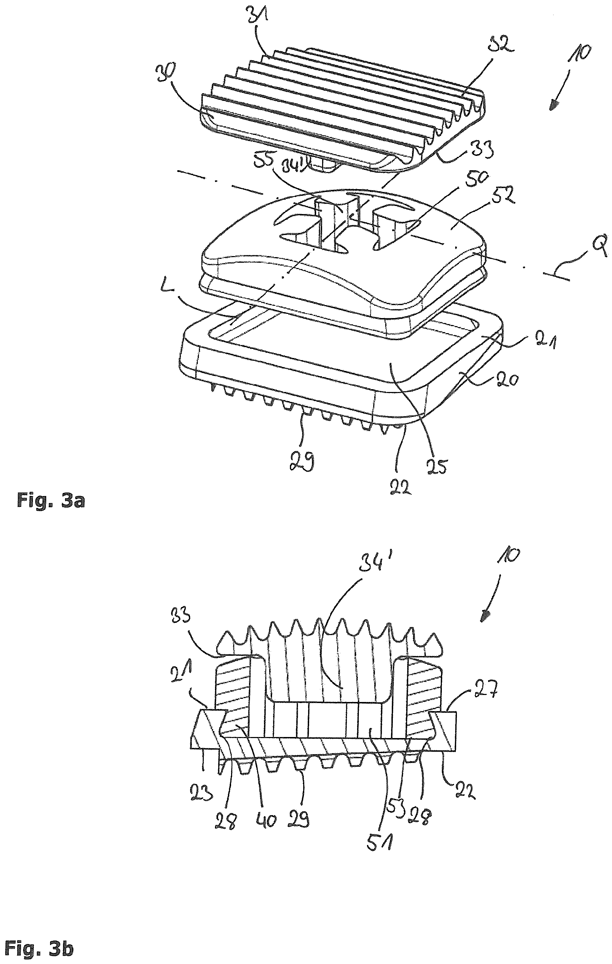

In den Fig. 3a bis 3c ist eine weitere Ausführungsform einer Bandscheibenprothese 10 dargestellt. Diese besteht wiederum aus einer kaudalen Platte 20, einer kranialen Platte 30 und einem elastischen Kern 40.

Insbesondere in der Fig. 3b ist zu erkennen, dass auch in diesem Ausführungsbeispiel der elastische Kern 40 in eine Ausnehmung 25 der kaudalen Platte 20 eingespritzt ist, wobei die Ausnehmung 25 wiederum eine vollumfänglich ausgebildete Hinterschneidung 28 aufweist.

Die Unterschiede sind insbesondere in der Form des Führungsstiftes 34' der kranialen Platte 30 sowie in der Formgebung der Führungskontur 50 des elastischen Kerns 40 zu erkennen. Die Führungskontur 50 weist im Wesentlichen eine Kreuzform auf. Der Führungsstift 34' weist eine hierzu komplementäre Form auf. Die Führungskontur 50 weist einen größeren Querschnitt als der Führungsstift 34' auf, so dass sich der Führungsstift 34' innerhalb der Führungskontur 50 bewegen kann. Wie der Fig. 3b zu entnehmen ist, ist die Ausnehmung 51 der Führungskontur 50 über die gesamte Höhe des elastischen Kerns 40 ausgebildet. Der elastische Kern 40 ist wiederum sowohl in Längsrichtung L als auch in Querrichtung Q konvex gewölbt.

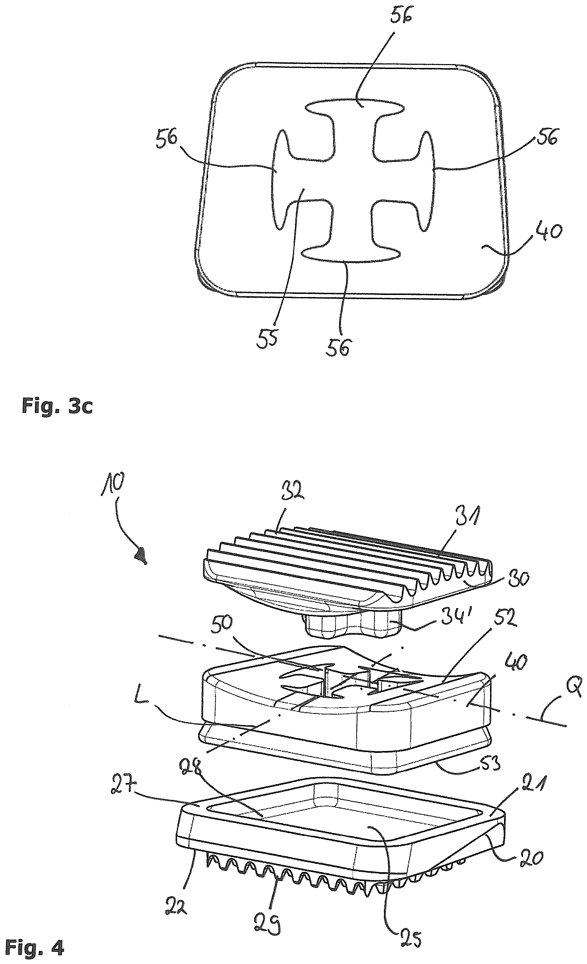

In Fig. 3c ist die Form der Führungskontur 50 und somit die Form des komplementär ausgebildeten Führungsstiftes 34' näher dargestellt. An die Kreuzform 55 schließen sich Querstege 56 an. Die Querstege 56 sind wiederum leicht gewölbt ausgebildet.

Die Führungskonturen 50 und/oder die Kernmitte 41 (dies betrifft die Ausführungsbeispiele der Fig. 1a bis 2b) bilden eine Art Neutralzone. Da die Ausnehmungen 51 der Führungskonturen 50 größer als die eingreifende Geometrie der Führungsstifte 34 und 34' sind, wird im Zusammenspiel mit der kranialen Platte 30 eine Bewegung erlaubt, wobei die kraniale Platte 30 im elastischen Kern 40 geführt wird.

Die Dimension des Bewegungsumfangs in der Neutralzone wird über die Geometrie der Führungskontur 50 sowie der zugehörigen Ausnehmung 51 abgebildet. In der Neutralzone wird die Bewegung mechanisch über die Geometrie des elastischen Kerns 40 geführt und mit geringster Last bzw. Gegenkraft durchgeführt. Aufgrund der Geometrie der Ausnehmung 51 bzw. der Führungskontur 50 im Kern 40 und der invers gestalteten Geometrie des Führungsstiftes 34 bzw. 34' der kranialen Platte 30 wird verhindert, dass es zu einer Dislokation der Bandscheibenprothese 10 kommt.

In Fig. 4 ist eine zusätzliche Ausführungsform einer Bandscheibenprothese 10 mit einem kreuzförmigen Führungsstift 34' und einer komplementär ausgebildeten Ausnehmung 51 der Führungskontur 50 dargestellt. In Fig. 4 ist der elastische Kern 40 sowohl in Querrichtung Q als auch in Längsrichtung L konkav gewölbt. Es ist auch denkbar, dass der elastische Kern 40, insbesondere die Seite 52 lediglich in Längsrichtung L oder lediglich in Querrichtung Q konkav geformt ist.

### Bezugszeichenliste

- 10: Bandscheibenprothese
- 20: Kaudale Platte
- 21: Seite kaudale Platte
- 22: Unterseite
- 23: Randabschnitt
- 25: Ausnehmung
- 27: Randbereich
- 28: Hinterschneidung
- 29: Riffelung
- 30: Kraniale Platte
- 31: Oberseite
- 32: Verzahnung
- 33: Seite kraniale Platte
- 34, 34': Führungsstift
- 35: Verbindungsabsatz
- 40: Elastischer Kern
- 41: Kernmitte
- 42: Umfangsabschnitt
- 45: Nut
- 50: Führungskontur
- 51: Ausnehmung
- 52: Seite
- 53: Unterseite
- 55: Kreuzform
- 56: Querstrebe
- L: Längsrichtung
- Q: Querrichtung

## Patentansprüche

1. Bandscheibenprothese (10), umfassend eine kaudale Platte (20), eine kraniale Platte (30) und einen zwischen der kaudalen Platte (20) und der kranialen Platte (30) ausgebildeten elastischen Kern (40),
wobei
die kaudale Platte (20) auf der zur kranialen Platte (30) weisenden Seite (21) eine Ausnehmung (25) aufweist, wobei die kraniale Platte (30) nicht mit dem Kern (40) verbunden ist,
**dadurch gekennzeichnet, dass**
der Kern (40) formschlüssig mit der Ausnehmung (25) der kaudalen Platte (20) verbunden ist, wobei die kraniale Platte (30) mindestens zwei Führungsstifte (34) aufweist, die in eine Führungskontur (50) des Kerns (40) beweglich eingreifen und die Führungskontur (50) des Kerns (40) als mindestens zwei gebogene, insbesondere nierenförmige, Ausnehmungen (51) ausgebildet ist.

2. Bandscheibenprothese (10), umfassend eine kaudale Platte (20), eine kraniale Platte (30) und einen zwischen der kaudalen Platte (20) und der kranialen Platte (30) ausgebildeten elastischen Kern (40),
wobei
die kaudale Platte (20) auf der zur kranialen Platte (30) weisenden Seite (21) eine Ausnehmung (25) aufweist, wobei die kraniale Platte (30) nicht mit dem Kern (40) verbunden ist,
**dadurch gekennzeichnet, dass**
der Kern (40) formschlüssig mit der Ausnehmung (25) der kaudalen Platte (20) verbunden ist, wobei die kraniale Platte (30) mindestens einen Führungsstift (34') aufweist, der in eine Führungskontur (50) des Kerns (40) beweglich eingreift und der Führungsstift (34') im Querschnitt im Wesentlichen eine Kreuzform aufweist und die Führungskontur (50) des Kerns (40) als eine komplementär zur Kreuzform ausgebildete Ausnehmung (51) ausgebildet ist.

3. Bandscheibenprothese (10) nach Anspruch 1 oder 2,
**dadurch gekennzeichnet, dass**
der Kern (40) in die Ausnehmung (25) eingespritzt oder eingegossen, insbesondere mittels eines Urformverfahrens, besonders bevorzugt mittels eines Kunststoffspritzgießverfahrens oder Vakuumgussverfahrens eingespritzt oder eingegossen, ist.

4. Bandscheibenprothese (10) nach Anspruch 1, 2 oder 3,
**dadurch gekennzeichnet, dass**
der Kern (40) viskoelastische Eigenschaften aufweist und/oder aus einem Elastomer, insbesondere aus Silikon oder PCU, besteht.

5. Bandscheibenprothese (10) nach einem der vorhergehenden Ansprüche,
**dadurch gekennzeichnet, dass**
der Kern (40) auf der zur kranialen Platte (30) weisenden Seite (52) gewölbt, insbesondere in Längsrichtung (L) und in Querrichtung (Q) gewölbt, ausgebildet ist.

6. Bandscheibenprothese (10) nach einem der vorhergehenden Ansprüche,
**dadurch gekennzeichnet, dass**
die zum Knochen weisende Seite (22) der kaudalen Platte (20) und/oder die zum Knochen weisende Seite (31) der kranialen Platte (30) eine Verzahnung (32) und/oder Riffelung (29) aufweist.

7. Bandscheibenprothese (10) nach einem der vorhergehenden Ansprüche,
**dadurch gekennzeichnet, dass**
in der Ausnehmung (25) der kaudalen Platte (20) zumindest abschnittsweise, insbesondere vollumfänglich, eine Hinterschneidung (28) ausgebildet ist.

8. Verfahren zur Herstellung einer Bandscheibenprothese (10), insbesondere einer Bandscheibenprothese nach einem der Ansprüche 1 bis 7,
**gekennzeichnet durch**
die Verfahrensschritte:
a) Bereitstellen einer kaudalen Platte (20) mit einer Ausnehmung (25), die vorzugsweise zumindest abschnittsweise eine Hinterschneidung (28) aufweist,
b) Einspritzen und/oder Eingießen eines elastischen, insbesondere viskoelastischen, Materials in die Ausnehmung (25) und Bilden eines elastischen, insbesondere viskoelastischen, Kerns (40).

9. Verfahren nach Anspruch 8,
**dadurch gekennzeichnet, dass**
die kaudale Platte (20) vor Schritt b) in eine Form, insbesondere in ein Spritzgusswerkzeug, eingelegt wird.

10. Verfahren nach Anspruch 8 oder 9,
**gekennzeichnet durch**
c) Auflegen einer kranialen Platte (30) auf den Kern (40) derart, dass mindestens ein Führungsstift (34, 34') der kranialen Platte (30) in eine Führungskontur (50) des Kerns (40) beweglich eingreift.

## Claims

1. An intervertebral disk prosthesis (10), comprising a caudal plate (20), a cranial plate (30), and an elastic core (40) formed between the caudal plate (20) and the cranial plate (30),
wherein
the caudal plate (20) has a cavity (25) on the side (21) facing the cranial plate (30), wherein the cranial plate (30) is not connected to the core (40),
**characterized in that**
the core (40) is connected positively to the cavity (25) of the caudal plate (20), wherein the cranial plate (30) has at least two guiding pins (34), which movably engage into the guiding contour (50) of the core (40), and the guiding contour (50) of the core (40) is formed as at least two bent, in particular kidney-shaped cavities (51).

2. An intervertebral disk prosthesis (10) comprising a caudal plate (20), a cranial plate (30), and an elastic core (40) formed between the caudal plate (20) and the cranial plate (30),
wherein
the caudal plate (20) has a cavity (25) on the side (21) facing the cranial plate (30), wherein the cranial plate (30) is not connected to the core (40),
**characterized in that**
the core (40) is connected positively to the cavity (25) of the caudal plate (20), wherein the cranial plate (30) has at least one guiding pin (34'), which movably engages into the guiding contour (50) of the core (40), and the guiding pin (34') essentially has a cross-shape in cross-section, and the guiding contour (50) of the core (40) is formed as a cavity that is formed to be complementary to the cross-shape of the cavity (51).

3. The intervertebral disk prosthesis (10) according to claim 1 or 2,
**characterized in that**
the core (40) is injected or cast into the cavity (25), in particular by means of a primary shaping method, particularly preferred is injected or cast in by means of a plastic injection molding method or vacuum casting method.

4. The intervertebral disk prosthesis (10) according to claim 1, 2 or 3,
**characterized in that**
the core (40) has viscoelastic properties and/or is made of an elastomer, in particular of silicone or PCU.

5. The intervertebral disk prosthesis (10) according to any one of the preceding claims,
**characterized in that**
the core (40) is formed to be arched on the side (52) facing towards the cranial plate (30), in particular is formed to be arched in the longitudinal direction (L) and the cross-direction (Q).

6. The intervertebral disk prosthesis (10) according to any one of the preceding claims,
**characterized in that**
the side (22) of the caudal plate (20) facing the bone and/or the side (31) of the cranial plate (30) facing the bone have/has a tooth system (32) and/or corrugation (29).

7. The intervertebral disk prosthesis (10) according to any one of the preceding claims,
**characterized in that**
in the cavity (25) of the caudal plate (20), an undercut (28) is formed at least in sections, in particular over the entire circumference.

8. A method for producing an intervertebral disk prosthesis (10), in particular an intervertebral disk prosthesis according to any one of claims 1 to 7,
**characterized by**
the method steps of:
a) providing a caudal plate (20) comprising a cavity (25) preferably having an undercut (28) at least in sections,
b) injecting or casting an elastic, in particular viscoelastic material into the cavity (25), and forming an elastic, in particular viscoelastic core (40).

9. The method according to claim 8,
**characterized in that**
prior to step b), the caudal plate (20) is put into a mold, in particular in an injection molding tool.

10. The method according to claim 8 or 9,
**characterized by**
c) applying a cranial plate (30) onto the core (40) such that at least one guiding pin (34, 34') of the cranial plate (30) movably engages into a guiding contour (50) of the core (40).

## Revendications

1. Prothèse de disque intervertébral (10), comprenant une plaque caudale (20), une plaque crâniale (30) et une âme (40) élastique constituée entre la plaque caudale (20) et la plaque crâniale (30),
sachant que
la plaque caudale (20) présente un évidement (25) du côté (21) tourné vers la plaque crâniale (30), sachant que la plaque crâniale (30) n'est pas reliée à l'âme (40),
**caractérisée en ce que**
l'âme (40) est reliée par complémentarité de forme à l'évidement (25) de la plaque caudale (20), sachant que la plaque crâniale (30) présente au moins deux pions de guidage (34) qui sont en prise de manière mobile dans un contour de guidage (50) de l'âme (40) et le contour de guidage (50) de l'âme (40) est constituée comme au moins deux évidements (51) courbés, en particulier en forme de reins.

2. Prothèse de disque intervertébral (10), comprenant une plaque caudale (20), une plaque crâniale (30) et une âme (40) élastique constituée entre la plaque caudale (20) et la plaque crâniale (30),
sachant que
la plaque caudale (20) présente un évidement (25) du côté (21) tourné vers la plaque crâniale (30), sachant que la plaque crâniale (30) n'est pas reliée à l'âme (40),
**caractérisée en ce que**
l'âme (40) est reliée par complémentarité de forme à l'évidement (25) de la plaque caudale (20), sachant que la plaque crâniale (30) présente au moins un pion de guidage (34') qui est en prise de manière mobile dans un contour de guidage (50) de l'âme (40) et le pion de guidage (34') présente sensiblement une forme de croix en coupe transversale et le contour de guidage (50) de l'âme (40) est constitué comme un évidement (51) constitué de manière complémentaire à la forme de croix.

3. Prothèse de disque intervertébral (10) selon la revendication 1 ou 2,
**caractérisée en ce que**
l'âme (40) est injectée ou moulée dans l'évidement (25), en particulier moyennant un procédé de formage primaire, en particulier de préférence est injectée ou moulée moyennant un procédé de moulage par injection de plastique ou un procédé de coulée sous vide.

4. Prothèse de disque intervertébral (10) selon la revendication 1, 2 ou 3,
**caractérisée en ce que**
l'âme (40) présente des propriétés viscoélastiques et/ou est composée d'un élastomère, en particulier de silicone ou de PCU.

5. Prothèse de disque intervertébral (10) selon l'une des revendications précédentes,
**caractérisée en ce que**
l'âme (40) est constituée, du côté (52) tourné vers la plaque crâniale (30), de manière voûtée, en particulier voûtée en direction longitudinale (L) et en direction transversale (Q).

6. Prothèse de disque intervertébral (10) selon l'une des revendications précédentes,
**caractérisée en ce que**
le côté (22) tourné vers l'os de la plaque caudale (20) et/ou le côté (31) tourné vers l'os de la plaque crâniale (30) présente une denture (32) et/ou une ondulation (29).

7. Prothèse de disque intervertébral (10) selon l'une des revendications précédentes,
**caractérisée en ce que**
un dégagement (28) est constitué, du moins par sections, en particulier sur toute la circonférence, dans l'évidement (25) de la plaque caudale (20).

8. Procédé de fabrication d'une prothèse de disque intervertébral (10), en particulier d'une prothèse de disque intervertébral selon l'une des revendications 1 à 7,
**caractérisé par**
les étapes de procédé :
a) mise à disposition d'une plaque caudale (20) avec un évidement (25) qui présente de préférence, du moins par sections, un dégagement (28),
b) injection et/ou moulage d'un matériau élastique, en particulier viscoélastique, dans l'évidement (25) et formation d'une âme (40) élastique, en particulier viscoélastique.

9. Procédé selon la revendication 8,
**caractérisé en ce que**
la plaque caudale (20) est insérée avant l'étape b) dans un moule, en particulier dans un outil de moulage par injection.

10. Procédé selon la revendication 8 ou 9,
**caractérisé par**
c) pose d'une plaque crâniale (30) sur l'âme (40) de telle manière qu'au moins un pion de guidage (34, 34') de la plaque crâniale (30) soit en prise de manière mobile dans un contour de guidage (50) de l'âme (40).
